# EUROPEAN PATENT APPLICATION

(11) **EP 4 461 327 A1**
(43) Date of publication of application: **13.11.2024**
(21) Application number: 22919020.2
(22) Date of filing: 02.12.2022
(51) Int. Cl.: A61M 5/142, A61M 5/145, A61M 5/315

(54) **LIQUID MEDICINE INJECTION APPARATUS**

(30) Priority: 04.01.2022 KR 20220001044; 04.02.2022 KR 20220014998
(71) Applicant: Eoflow Co., Ltd., Bundang-gu, Seongnam-si Gyeonggi-do 13605 (KR)
(72) Inventor: KIM, Chang Jung, Seongnam-si Gyeonggi-do 13588 (KR); LEE, Do Kyung, Hwaseong-si Gyeonggi-do 18466 (KR)
(74) Representative: AWA Sweden AB
(86) International application number: PCT/KR2022/019434
(87) International publication number: WO 2023/132483

(57) **Abstract**

An apparatus for infusing medical liquid may include: a base body, a needle assembly mounted on the base body, a reservoir unit fluidly connected to the needle assembly and having a plunger therein, a driving unit including a rod, which is connected to the plunger and moves along the reservoir unit, and a driving wheel, which has a protrusion and transmits driving force to the rod, and a connection member disposed between the rod and the driving wheel and having a guide groove for guiding the movement of the protrusion.

## Description

### Technical Field

The present disclosure relates to an apparatus for infusing medical liquid.

### Background Art

In general, apparatuses for infusing medical liquid such as apparatuses for infusing insulin are used to inject medical liquid into a patient's body. These apparatuses for infusing medical liquid are sometimes used by medical professionals such as doctors and nurses, but in most cases, they are used by the general public such as the patients themselves or guardians.

Diabetic patients, especially childhood diabetic patients, need to inject medical liquid such as insulin into a body at set intervals. A patch-type apparatus for infusing medical liquid that is attached to a human body for a set period is being developed, and this apparatus for infusing medical liquid can be used by attaching a patch to a human body, such as a patient's abdomen or waist, for a set period.

To increase the effect through infusing medical liquid, an apparatus for infusing medical liquid needs to be controlled to precisely inject medical liquid into a patient's body. It is important to precisely inject a small amount of medical liquid through a small apparatus for infusing medical liquid.

When attached to a human body, an apparatus for infusing medical liquid needs to be comfortable to wear, convenient to use, durable, and driven at low power. In particular, since an apparatus for infusing medical liquid is used by attaching it directly to a patient's skin, it is important for a user to conveniently and safely drive the apparatus for infusing medical liquid.

### Disclosure

### Technical Problem

The present disclosure provides an apparatus for infusing medical liquid that drives safely and can precisely deliver drugs.

### Technical Solution

A liquid medicine injection apparatus may comprise: a base body, a needle assembly mounted on the base body, a reservoir unit fluidly connected to the needle assembly and having a plunger therein, a driving unit including a rod, which is connected to the plunger and moves along the reservoir unit, and a driving wheel, which has a protrusion and transmits driving force to the rod, and a connection member disposed between the rod and the driving wheel and having a guide groove for guiding the movement of the protrusion.

### Advantageous Effects of the Disclosure

An apparatus for infusing medical liquid and the driving method thereof according to the present disclosure can be driven simply and safely by a user. When the user rotates a sleeve of a needle assembly, a cannula is inserted into an object and a preparation for infusing medical liquid is completed. At the same time, a connection member connects a driving unit and a driving module to allow medical liquid to be injected from a reservoir to the needle, and the driving module is started driving. Therefore, the user can safely start driving an apparatus for infusing medical liquid by simply rotating the needle assembly.

An apparatus for infusing medical liquid according to the present disclosure and the driving method thereof may inject a safe and fixed amount of drugs into the object, because after the cannula of the needle assembly is inserted into the object, a structure for discharging medical liquid is driven and connected by a connection member.

An apparatus for infusing medical liquid according to the present disclosure and the driving method thereof may ensure user safety, because the driving is started intuitively by rotating the needle assembly, and through this, it is possible to confirm whether the driving module and the driving unit are driven normally, and accordingly the coupling of the driving unit and the connection member may be performed efficiently without any failure. Of course, the field of the present disclosure is not limited by these effects.

### Brief Description of Drawings

FIG. 1 is a perspective view showing an apparatus for infusing medical liquid according to an embodiment of the present disclosure.
FIG. 2 is a perspective view showing an internal arrangement of the apparatus for infusing medical liquid of FIG. 1.
FIG. 3 is a perspective view showing a coupling relationship between a driving unit and a connection member.
FIG. 4 is a cross-sectional diagram showing the coupling relationship between the driving unit and the connection member of FIG. 3.
FIGS. 5 and 6 are perspective views showing a coupling relationship between a driving unit and a connection member.
FIGS. 7 to 10 are cross-sectional diagrams showing the driving of injecting medical liquid into a reservoir, storing the medical liquid, and discharging the medical liquid through a needle.
FIG. 11 is a flow chart showing a method of driving method of an apparatus for infusing medical liquid according to other embodiment of the present disclosure.
FIG. 12 is a flow chart showing some steps in FIG. 11.

### Best Mode for Implementing the Disclosure

One aspect of the present disclosure provides an apparatus for infusing medical liquid including a base body, a needle assembly mounted on the base body, a reservoir unit fluidly connected to the needle assembly and having a plunger therein, a driving unit including a rod, which is connected to the plunger and moves along the reservoir unit, and a driving wheel, which has a protrusion and transmits driving force to the rod, and a connection member disposed between the rod and the driving wheel and having a guide groove for guiding the movement of the protrusion.

Additionally, the connection member may have a trap groove extending from the guide groove in a circumferential direction of the connection member.

Additionally, the trap groove may be extended along a rotational direction of the driving wheel.

Additionally, the trap groove may be disposed in a plural number along a lengthwise direction of the connection member.

Additionally, when medical liquid is injected into the reservoir unit, the connection member may be inserted into the inside of the driving wheel while passing through the protrusion.

Additionally, when the driving wheel rotates, the protrusion is caught by the trap groove, and the driving wheel and the connection member may be coupled to each other.

Additionally, when the driving wheel rotates further, the connection member and the driving wheel rotate together, and the rod may move forward.

Additionally, the trap groove is disposed at a portion connected to the guide groove and may include a first side wall guiding the movement of the protrusion, and a second side wall connected to the first side wall and supporting the protrusion.

Additionally, the connection member may include a first portion adjacent to the plunger, and a second portion connected to the first portion and having a smaller diameter than the first portion, and the guide groove may be disposed at the first portion.

Additionally, the protrusion may protrude from an inner peripheral surface of the driving wheel.

### Mode For Implementing the Disclosure

Since the present disclosure can be modified in various ways and may have various embodiments, specific embodiments will be illustrated in diagrams and described in detail in a detailed description. The effects and features of the present disclosure and the methods for achieving thereof will become clear with reference to the embodiments described in detail below along with the diagrams. However, the present disclosure is not limited to the embodiments disclosed below and may be implemented in various forms.

Hereinafter, the embodiments of the present disclosure will be described in detail with reference to the accompanying diagrams and when describing with reference to the diagrams, identical or corresponding components will be assigned the same reference numerals, and the redundant description thereof will be omitted.

In the following embodiments, singular terms include plural terms, unless the context clearly dictates otherwise.

In the following embodiments, terms such as include or have mean the presence of features or components described in the specification, and do not exclude in advance the possibility of adding one or more other features or components.

In cases where an embodiment may be implemented differently, a specific process order may be performed differently from the described order. For example, two processes described in succession may be performed substantially at the same time or may be performed in an order opposite to that in which they are described.

In the diagrams, the sizes of components may be exaggerated or reduced for convenience of description. For example, the size and thickness of each component shown in the diagrams are arbitrarily shown for convenience of description, so the following embodiments are not necessarily limited to what is shown.

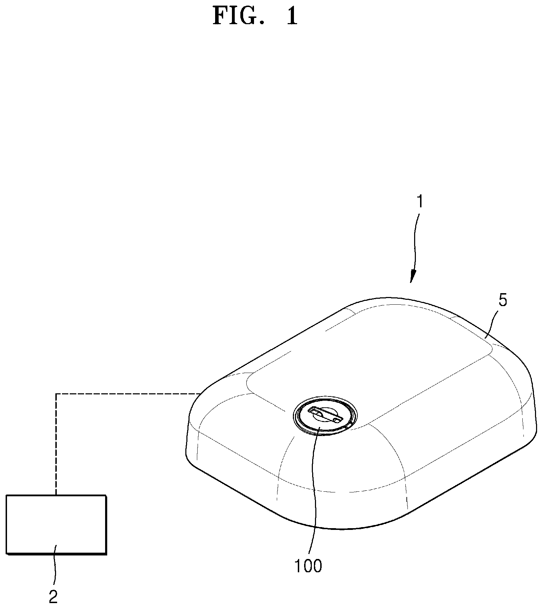

FIG. 1 is a perspective view showing an apparatus for infusing medical liquid 1 according to an embodiment of the present disclosure.

Referring to FIG. 1, the apparatus for infusing medical liquid 1 is attached to an object into which medical liquid is injected and may inject a user with a fixed amount of medical liquid stored inside. In an optional embodiment, the apparatus for infusing medical liquid 1 may be mounted on a user's body. Additionally, in another optional embodiment, the apparatus for infusing medical liquid 1 may be mounted on an animal to inject medial liquid.

The apparatus for infusing medical liquid 1 may be used for various purposes depending on the type of medical liquid to be injected. For example, the medical liquid may include medical liquid based on insulin for diabetic patients, medical liquid for pancreas, medical liquid for heart, and other various types of medical liquid.

The apparatus for infusing medical liquid 1 may be connected to a remote device 2 that is wired or wirelessly connected. The user may use the apparatus for infusing medical liquid 1 by manipulating the remote device 2 and monitor usage status of the apparatus for infusing medical liquid 1.

For example, the amount of medical liquid injected from the apparatus for infusing medical liquid 1, the number of injection of medical liquid, the amount of medical liquid stored in a reservoir unit 200, and user's bio-information may be monitored, and based on this, the user may drive the apparatus for infusing medical liquid.

In an embodiment, the remote device 2 refers to a communication terminal with which an application may be used in a wired or wireless communication environment.

Here, the remote device 2 may be a user's portable terminal. To explain this in more detail, the remote device 2 may include a computer (e.g. desktop, laptop, tablet), a media computing platform (e.g. cable, satellite set-top box, digital video recorder), or a handheld computing device (e.g. PDA, email client), any type of cell phone, any type of wearable device that may be used by attaching or mounting it to a user's body, or any other type of computing or communication platform, but the present disclosure is not limited thereto.

The apparatus for infusing medical liquid 1 and the remote device 2 may communicate through a communication network. At this time, the communication network refers to a communication network that provides a connection path so that the remote device 2 may transmit and receive data after connecting to a service server (not shown). The communication network may include, for example, wired networks such as LANs (Local Area Networks), WANs (Wide Area Networks), MANs (Metropolitan Area Networks), and ISDNs (Integrated Service Digital Networks), or wireless networks such as wireless LANs, CDMA, Bluetooth, and satellite communications, but the field of the present disclosure is not limited thereto.

According to FIG. 1, the remote device 2 is shown as a single device, but the present disclosure is not necessarily limited thereto and may include a plurality of devices capable of communicating with the apparatus for infusing medical liquid 1.

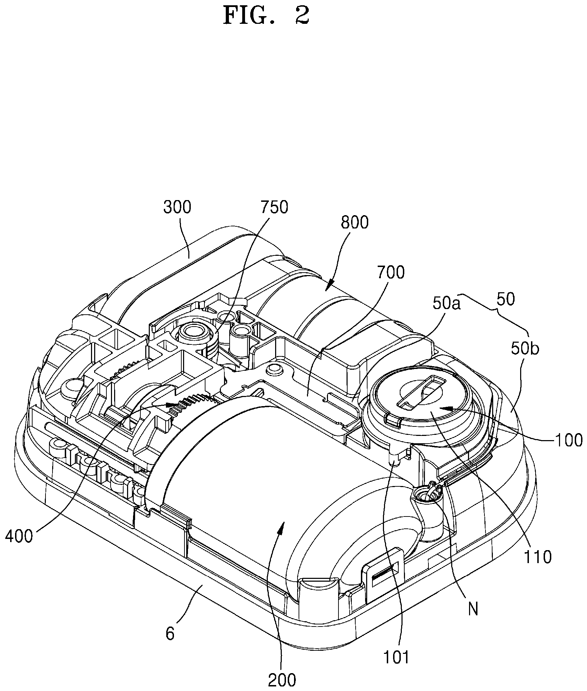

FIG. 2 is a perspective view showing the internal arrangement of an embodiment of the apparatus for infusing medical liquid 1 of FIG. 1.

Referring to FIGS. 1 and 2, an embodiment of the apparatus for infusing medical liquid 1 may include a housing 5 covering the outside and an attachment portion 6 located adjacent to a user's skin. The apparatus for infusing medical liquid 1 includes a plurality of parts disposed in an interior space between the housing 5 and the attachment portion 6. A separate joining means may be further interposed between the attachment portion 6 and the user's skin, and the apparatus for infusing medical liquid 1 may be fixed to the skin by the joining means.

The apparatus for infusing medical liquid 1 may include a base body 50, a needle assembly 100, a reservoir unit 200, a driving unit 400, and a connection member 500. Furthermore, the apparatus for infusing medical liquid 1 may further include a driving module 300, a trigger member 700, and a battery 800. Meanwhile, the driving unit 400 and the connection member 500 may be collectively referred to as a driving assembly 600.

The base body 50 forms a basic frame of the housing 5 and is mounted in the interior space of the housing 5. The base body 50 may be provided in a plural number. In an embodiment, a first body 50a covering an upper side of internal parts, and a second body 50b covering a lower side of internal parts may be provided. The first body 50a and the second body 50b may be assembled to fix the internal parts of the apparatus for infusing medical liquid 1 at a preset position. In another embodiment, the base body 50 may be formed as an integrated frame.

The needle assembly 100 may be mounted on the base body 50. A needle N and/or a cannula of the needle assembly 100 may be moved in an axial direction by the rotation of a sleeve 110.

One end of the needle N is connected to the reservoir unit 200 so that medical liquid may be delivered, and the other end is inserted into the cannula and may move along the cannula.

Since the cannula has a conduit shape capable of accommodating a needle N, the medical liquid discharged from the needle N may be injected into a user.

In the apparatus for infusing medical liquid 1, the user may simply rotate the needle assembly 100, insert the cannula into the object, and start an injection of the medical liquid. In detail, when the user first rotates the sleeve 110, the cannula is inserted into the object, and when the user rotates the sleeve 110 a second time, the needle N may be extracted from the object. At this time, with only the cannula inserted into the object, a knob 101 applies one end of the trigger member 700 to drive the driving module 300 to deliver medical liquid, and the medical liquid may be discharged quantitatively from the reservoir unit 200. Therefore, the user may conveniently and stably use the apparatus for infusing medical liquid 1.

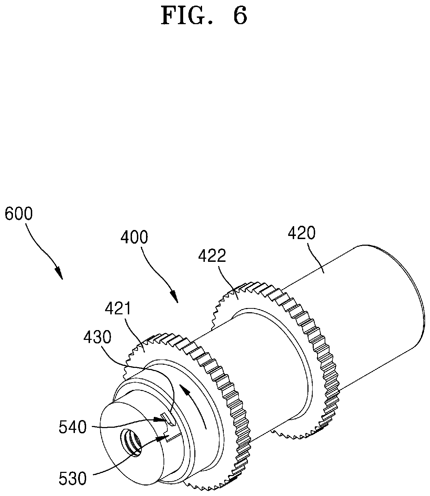

The reservoir unit 200 is mounted on the base body 50 and may be connected to the needle assembly 100. The reservoir unit 200 may include a reservoir 210, a cap cover 220, a plunger 230, and a sealing ring 240 (see FIG. 6).

The reservoir 210 may be extended to a preset length in a lengthwise direction to store medical liquid in an interior space. The reservoir 210 may discharge the medical liquid to the needle N by moving the plunger 230. The cap cover 220 is mounted on the end of the reservoir 210, and a rod 410 and/or the connection member 500 may be moved through an opening 225 disposed in the cap cover 220 (see FIG. 6).

The reservoir 210 may have an inlet end and an outlet end. Medical liquid is injected into the inlet end, a needle N is installed at the outlet end, and the medical liquid may be discharged through the needle N.

The plunger 230 is disposed inside the reservoir 210 and may move linearly by driving a driving module 300 and a driving unit 400. As the plunger 230 moves forward, the medical liquid may be discharged from the interior space to the needle N.

The plunger 230 may have an end 231 and an inclined surface 232. The end 231 may move toward a front (21 0F) of the reservoir 210 to move the medical liquid. The inclined surface 232 may be in close contact with an inclined portion of the reservoir 210.

The plunger 230 is provided with the sealing ring 240 at a portion in contact with an inside wall of the reservoir 210, thereby preventing a leakage of the medical liquid when the plunger 230 is moved.

The driving module 300 may generate driving force and transmit the driving force to the driving unit 400. The driving force transmitted by the driving unit 400 can linearly move the plunger 230 inside the reservoir 210 to discharge the medical liquid.

When the driving units 400 are engaged with each other by the connection member 500, the driving module 300 may rotate the driving wheel 420 of the driving unit 400, and by rotating the driving wheel 420, the rod 410 may move linearly to move the plunger 230.

The driving module 300 may be provided with any type of device having a suction power of medical liquid and a discharge power thereof by electricity. For example, all types of pumps, such as mechanical displacement micropumps and electromagnetic motion micropumps, may be used. A mechanical displacement micropump is a pump that uses movement of solids or fluids, such as gears or diagrams, to create a pressure difference to induce flow of fluid, and includes a diaphragm displacement pump, a fluid displacement pump, a rotary pump, or the like. An electromagnetic micropump is a pump that uses energy in a form of electricity or magnetism directly to movement of fluids, and includes an electrohydrodynamic pump (EHD), an electroosmotic pump, and a magnetohydrodynamic pump, a electro wetting pump, or the like.

In an embodiment, the driving module 300 may be electrically connected to a remote device 2, and the driving module 300 may be operated by controlling the remote device 2.

The driving unit 400 is installed between the driving module 300 and the reservoir unit 200 and may move the plunger 230 disposed inside the reservoir 210 with the driving force generated by the driving module 300. However, the driving unit 400 may move the plunger 230 forward only when the rod 410 and the driving wheel 420 are coupled or connected by the connection member 500.

The rod 410 is connected to the plunger 230 and extended in one direction. The rod 410 is inserted into the opening of the cap cover 220, and the rod 410 may move in a lengthwise direction of the reservoir 210 to move the plunger 230.

The driving wheel 420 is driveably connected to the driving module 300 and may rotate by driving the driving module 300.

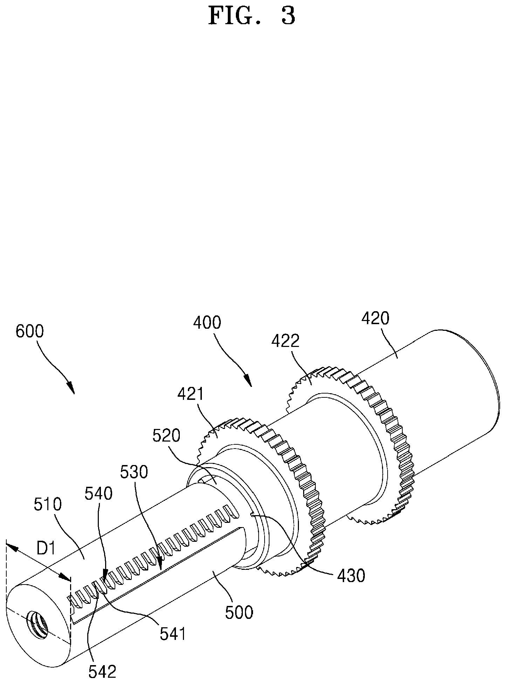

The driving wheel 420 may have a plurality of protrusions 430 at the inlet (see FIG. 3). The protrusions 430 may protrude from an inner peripheral surface of the driving wheel 420. In FIG. 3, the driving wheel 420 is shown as having three protrusions at the inlet, but the present disclosure is not necessarily limited thereto, and the driving wheel 420 may be provided with two or four or more protrusions at the inlet.

The driving wheel 420 has a first connection end 421 and a second connection end 422, and may have a space inside where the rod 410 and the connection member 500 may move. Since at least one of the first connection end 421 and the second connection end 422 is always driveably connected to the driving module 300 by a connector (not shown), so the driving wheel 420 may rotate.

In an embodiment, the first connection end 421 and the second connection end 422 may have a gear tooth shape. The connector connected to the driving module 300 may apply gear teeth to rotate the driving wheel 420.

In detail, the connector repeatedly rotates about an axis of rotation according to a linear reciprocating motion of the driving module 300. The end of the connector may rotate the driving wheel 420 by applying at least one of the first connection end 421 and the second connection end 422. For example, one end of the connector may be disposed to apply the first connection end 421, and the other end of the connector may be disposed to apply the second connection end 422.

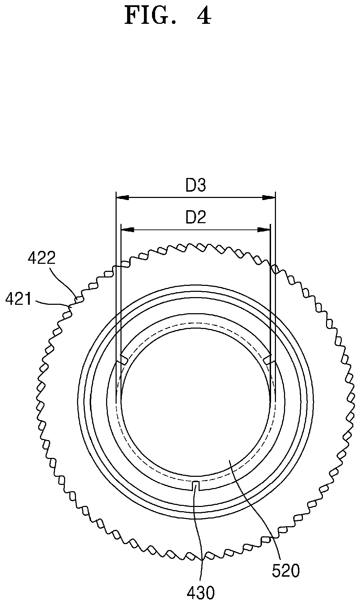

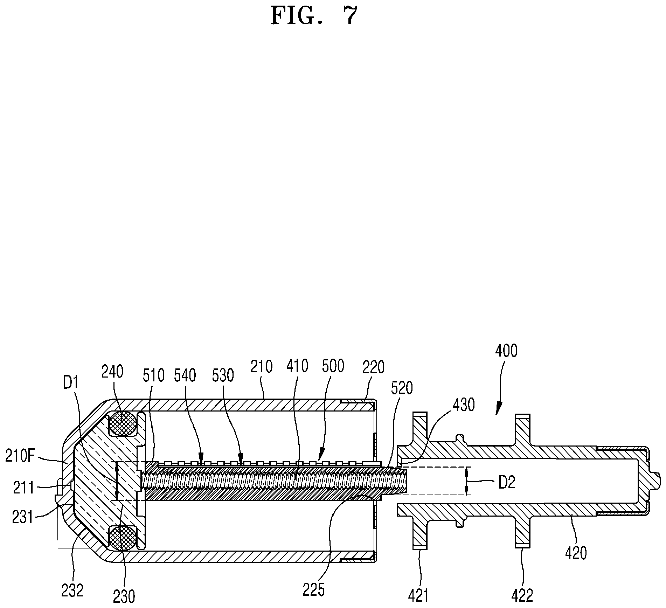

FIGS. 3, 5, and 6 are perspective views showing a coupling relationship between a driving unit and a connection member, FIG. 4 is a cross-sectional drawing showing the coupling relationship between the driving unit and the connection member of FIG. 3, and FIGS. 7 to 10 are cross-sectional views showing the driving of storing medical liquid injected into a reservoir and discharging the medical liquid through a needle.

Referring to FIGS. 3, 4, and 7, the connection member 500 may driveably connect the driving module 300 and the driving unit 400. The connection member 500 may be disposed between the rod 410 and the driving wheel 420.

The connection member 500 may include a first portion 510 located adjacent to the plunger 230 and having a first diameter D1 and a second portion 520 connected to the first portion 510 and having a second diameter D2.

The first portion 510 may be provided with a guide groove 530 that guides movement of the protrusions 430 and a trap groove 540 extended from the guide groove 530 in a circumferential direction of the connection member 500.

The second portion 520 may be extended from the guide groove 530, and accordingly, the second diameter D2 may be smaller than the first diameter D1.

In addition, the second diameter D2 may be smaller than a third diameter D3 formed by the protrusions 430 of the driving wheel 420, and accordingly, the connection member 500 and the driving wheel 420, which are in the case prior to an injection of medical liquid into the reservoir unit 200, may not be in contact with each other. Meanwhile, before medical liquid is injected into the reservoir unit 200, the driving wheel 420 may be tested using the driving force of the driving module 300. At this time, the second diameter D2 is smaller than the third diameter D3, so the driving wheel 420 may smoothly rotate without any contact with the connection member 500. That is, before medical liquid is injected, the second portion 520 of the connection member 500 may be overlapped with the driving wheel 420, but the second diameter D2 of the second portion 520 is smaller than the third diameter D3 of the driving wheel 420, so the second portion 520 and the driving wheel 420 may be not in contact with each other, and accordingly, it may be confirmed whether the driving wheel 420 is driving normally.

In addition, when the protrusions 430 and the guide groove 530 are not aligned in a lengthwise direction of the connection member 500, the driving wheel 420 may rotate without any contact with the connection member 500, so the protrusions 430 and the guide groove 530 may be aligned in a lengthwise direction of the connection member 500.

In an embodiment, the rod 410 and the connection member 500 may have the form of a screw and a thread, respectively. A thread is formed on an outer peripheral surface of the rod 410, and a thread is formed on an inner peripheral surface of the connection member 500, so that they may be connected in a screw-coupled form.

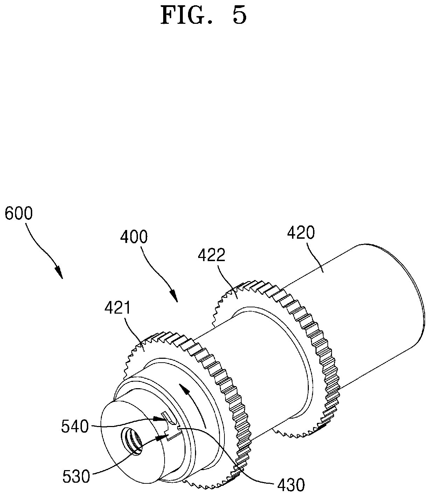

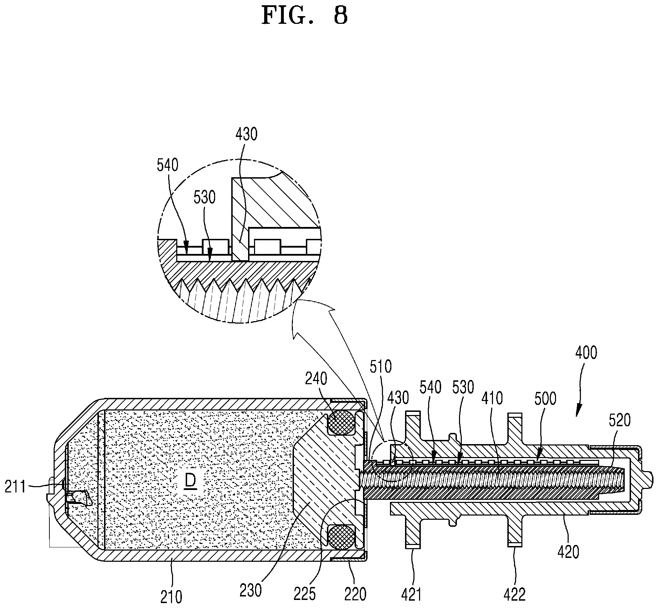

Referring to FIGS. 5 and 8, when medical liquid is injected into the reservoir unit 200, the connection member 500 may be inserted into an interior of the driving wheel 420 while passing through the protrusions 430. At this time, the guide groove 530 may guide movement of the protrusions 430.

The trap groove 540 may be formed in a plural number along a lengthwise direction of the connection member 500, and accordingly, even if the amount of medical liquid injected into the reservoir unit 200 varies, the protrusions 430 may be disposed to overlap at least one of the trap grooves 540 on the guide groove 530 in a rotational direction of the driving wheel 420.

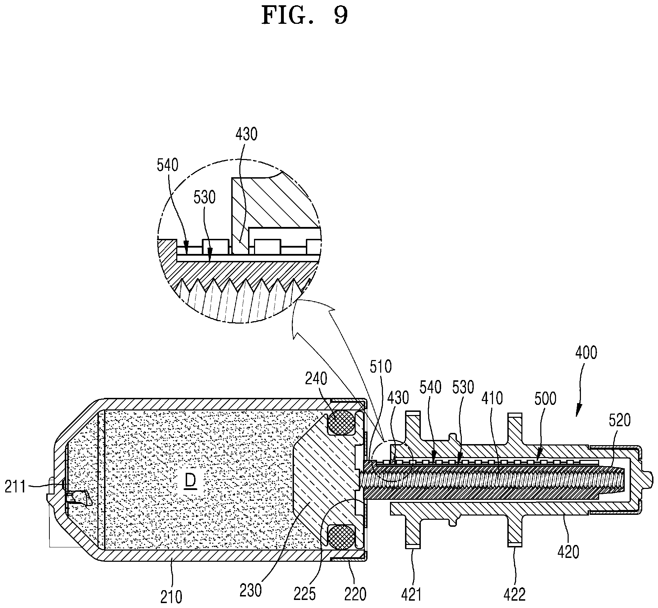

Referring to FIGS. 6 and 9, the driving wheel 420 of the driving unit 400 may be rotated using the driving force of the driving module 300. Since the trap groove 540 may be extended along a rotational direction of the driving wheel 420, when the driving wheel 420 rotates, the protrusions 430 are caught by the trap groove 540, and at this time, the driving wheel 420 and the connection member 500 may be coupled to each other.

In an embodiment, the trap groove 540 is disposed in a portion connected to the guide groove 530 and may include a first side wall 541 guiding the movement of the protrusions 430 and a second side wall 542 connected to the first side wall and supporting the protrusions 430. Accordingly, even though the protrusions 430 are disposed to overlap portions between the trap grooves 540 of the connection members 500 located adjacent to each other in a rotational direction of the driving wheel 420, when the driving wheel 420 rotates, the protrusions 430 may move under the guidance of the first wall 541 instead of being caught between the trap grooves 540 and may be caught by the trap groove 540 while being supported by the second side wall 542.

In an embodiment, both side walls of the protrusion 430 may be perpendicular to an inner peripheral surface of the driving wheel 420, and the side walls of the trap groove 540 may be perpendicular to an outer peripheral surface of the connection member 500. Accordingly, while the driving wheel 420 rotates, the protrusions 430 do not go over the trap groove 540 in a rotational direction but is accurately caught on the catching portion of the trap groove 540, thereby the coupling of the driving unit 400 and the connection member 500 may not be undone.

That is, the coupling of the driving unit 400 and the connection member 500 may be performed by rotating the driving wheel 420 through driving force of the driving module 300. As described above, before injecting medical liquid, the driving unit 400 may be tested using driving force of the driving module 300, and at this time, if the driving wheel 420 rotates smoothly without any contact with the connection member 500, it may be confirmed whether the driving unit 400 is driving normally. That is, with just the confirmation of normal drives of the driving module 300 and the driving unit 400 without intervention of other parts other than the driving module 300 and the driving unit 400, an operation of the driving assembly 600, or the coupling of the driving unit 400 and the connection member 500 may be performed efficiently without any failure.

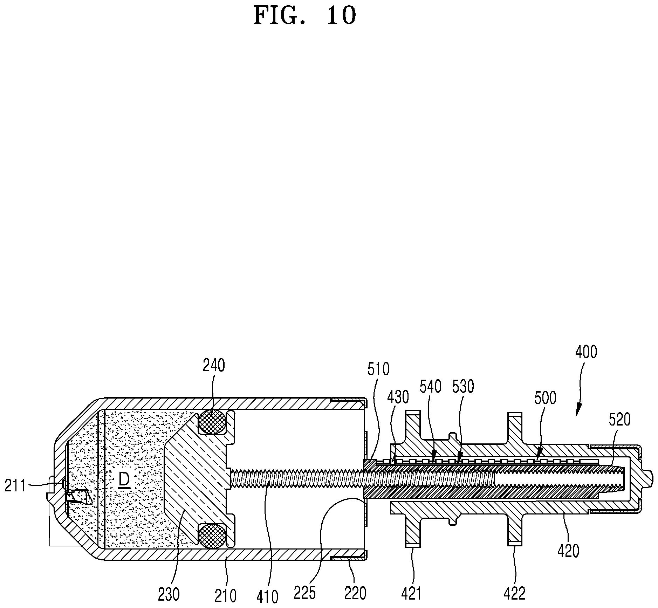

Referring to FIG. 10, when the driving wheel 420 rotates further, the connection member 500 may also rotate, and at this time, the rod 410 may move forward. That is, since the rod 410 and the connection member 500 are screw-coupled, the connection member 500 may move the rod 410 forward even if the driving wheel 420 rotates with a small torque. That is, the plunger 230 may move forward even with a slightly weak force due to the screw coupling of the rod 410 and the connection member 500.

Referring again to FIG. 2, the trigger member 700 may generate a mechanical signal that causes medical liquid of the apparatus for infusing medical liquid 1 to be injected. The trigger member 700 is rotatably disposed on one side of the base body 50, and the trigger member 700 may rotate to start driving the driving module 300.

The other end of the trigger member 700 extends to the driving module 300, and a starter 750 may maintain a driving shaft of the driving module 300 in a fixed state by the other end of the trigger member 700.

In detail, when the user rotates the needle assembly 100, the knob 101 of the needle assembly 100 may apply the one end of the trigger member 700 to start the rotation of the trigger member 700. When the trigger member 700 rotates, the starter 750 releases contact with the other end of the trigger member 700 and applies the driving shaft to start driving the driving module 300.

In an embodiment, the starter 750 may be formed in the form of a spring having a preset elastic force, but it is not limited thereto and may be formed in the form of a stick having a predetermined elasticity.

One end of the starter 750 is mounted on the base body 50, and the other end is disposed to support the trigger member 700. One end of the starter 750 is disposed between the end of the driving shaft and the other end of the trigger member 700.

A battery 800 may supply power to the apparatus for infusing medical liquid 1. The battery 800 is connected to the driving module 300 and may control driving of the driving module 300. The battery 800 may be rechargeable or disposable.

In a diagram, a pair of batteries 800 are shown, but they are not limited thereto, and may be set in various ways depending on the capacity, usage range, usage time, or the like of the apparatus for infusing medical liquid 1.

The apparatus for infusing medical liquid 1 according to an embodiment of the present disclosure may be driven simply and safely by a user. When the user rotates the sleeve 110 of the needle assembly 100, the cannula is inserted into the object, the connection member 500 connects the driving unit 400, thereby injecting medical liquid with a driving force of the driving module 300, and at the same time, the driving module 300 is started driving, so the apparatus for infusing medical liquid 1 may be used simply and safely.

The apparatus for infusing medical liquid 1 according to an embodiment of the present disclosure may inject a safe and fixed amount of drugs into an object, because after the cannula of the needle assembly 100 is inserted into the object, a structure for discharging medical liquid is driven and connected by the connection member 500.

The apparatus for infusing medical liquid 1 according to an embodiment of the present disclosure is intuitively started driving through the rotation of the needle assembly 100, so it may be confirmed whether the driving module 300 and the driving unit 400 are driving normally, and accordingly, an operation of the coupling of the driving unit 400 and the connection member 500, or the driving assembly 600, may be performed efficiently without any failure, thereby ensuring user safety.

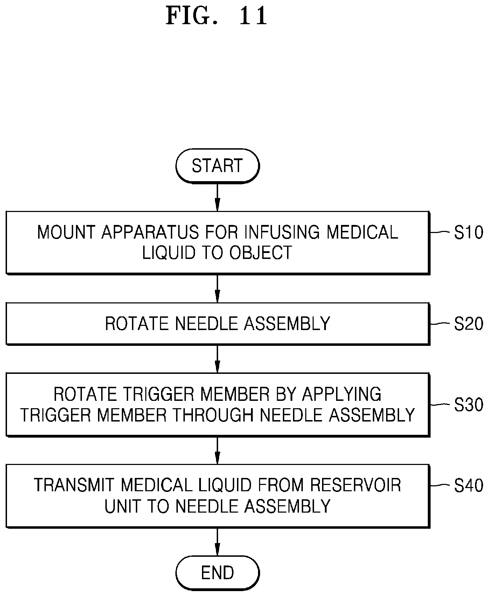

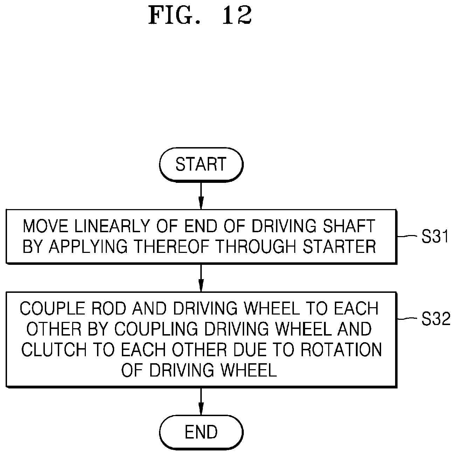

FIG. 11 is a flow chart showing a method of driving an apparatus for infusing medical liquid according to another embodiment of the present disclosure, and FIG. 12 is a flow chart showing some steps of FIG. 11.

Referring to FIGS. 11 and 12, the method of driving the apparatus for infusing medical liquid 1 may include a step (S10) of mounting an apparatus for infusing medical liquid on an object, a step (S20) of rotating the needle assembly 100, a step (S30) of rotating a trigger member by applying the trigger member through the needle assembly, and a step (S40) of transmitting medical liquid from a reservoir to the needle assembly.

In the step (S10) of mounting an apparatus for infusing medical liquid on an object, an attachment portion 6 of the apparatus for infusing medical liquid 1 is attached to an object. Additionally, it can be connected to the remote device 2 in a wired or wireless communication environment.

In the step (S20) of rotating the needle assembly 100, the needle assembly 100 is driven by a user. As described above, when the sleeve 110 of the needle assembly 100 is first rotated by the user, the cannula and the needle N are inserted into a skin together, and when the sleeve 110 is rotated a second time, only needle N is withdrawn from the skin. The first rotation of the sleeve 110 is to secure the cannula to the skin using the needle N, and the second rotation is to withdraw the needle N and inject medical liquid.

The step (S30) of rotating a trigger member 700 by applying the trigger member 700 through the needle assembly 100 occurs simultaneously with the rotation of the needle assembly 100. As the needle assembly 100 rotates, the knob 101 applies the one end of the trigger member 700.

At this time, a step (S31) occurs in which the starter 750 applies the end of the driving shaft and the end of the driving shaft moves linearly. As the contact between the starter 750 and the other end of the trigger member 700 is released, the starter 750 may start driving the driving module 300. As the starter 750 applies and passes through the end of the driving shaft, the end moves linearly along an axis. Thereafter, the driving module 300 linearly moves the driving shaft repeatedly, so driving force may be transmitted to the driving unit 400.

Thereafter, a step (S32) occurs in which the driving wheel 420 and the connection member 500 are coupled to each other as the driving wheel 420 rotates, thereby coupling the rod 410 and the driving wheel 420.

When the driving wheel 420 rotates, the protrusions 430 are caught by the trap groove 540, and at this time, the driving wheel 420 and the connection member 500 may be coupled to each other.

In the step (S40) of transmitting medical liquid from a reservoir to the needle assembly 100, an electrochemical reaction is generated in the driving module 300, and the medical liquid of the reservoir unit 200 may be delivered to an object by repetitive linear movement of the driving shaft of the driving module 300.

The spirit of the present disclosure should not be limited to the embodiments described above, and not only the claims described below, but also all fields equal to or equivalently changed from the claims fall within the field of the spirit of the present disclosure.

## Claims

1. An apparatus for infusing medical liquid, the apparatus comprising:
a base body,
a needle assembly mounted on the base body,
a reservoir unit fluidly connected to the needle assembly and having a plunger therein,
a driving unit including a rod that is connected to the plunger and moves along the reservoir unit, and a driving wheel that transmits a driving force to the rod and has a protrusion, and
a connection member disposed between the rod and the driving wheel and having a guide groove that guides movement of the protrusion.

2. The apparatus for infusing medical liquid of claim 1,
wherein the connection member
comprises a trap groove extending from the guide groove in a circumferential direction of the connection member.

3. The apparatus for infusing medical liquid of claim 2,
wherein the trap groove extends in a rotational direction of the driving wheel.

4. The apparatus for infusing medical liquid of claim 2,
wherein the trap groove is disposed in a plural number along a lengthwise direction of the connection member.

5. The apparatus for infusing medical liquid of claim 1,
wherein when medical liquid is injected into the reservoir unit,
the connection member is inserted into an interior of the driving wheel while passing through the protrusion.

6. The apparatus for infusing medical liquid of claim 2,
wherein when the driving wheel rotates, the protrusion is caught by the trap groove, thereby coupling the driving wheel and the connection member to each other.

7. The apparatus for infusing medical liquid of claim 6,
wherein when the driving wheel rotates further, the connection member and the driving wheel rotate together to move the rod forward.

8. The apparatus for infusing medical liquid of claim 2,
wherein the trap groove
has a first side wall disposed at a portion thereof connected to the guide groove and guiding movement of the protrusion and
a second side wall connected to the first side wall and supporting the protrusion.

9. The apparatus for infusing medical liquid of claim 1,
wherein the connection member has
a first portion adjacent the plunger and
a second portion connected to the first portion and having a smaller diameter than the first part, wherein the guide groove is disposed at the first portion.

10. The apparatus for infusing medical liquid of claim 1,
wherein the protrusion protrudes from an inner peripheral surface of the driving wheel.
